# EUROPEAN PATENT SPECIFICATION

(11) **EP 3 003 285 B1**
(45) Date of publication and mention of the grant of the patent: **16.02.2022**
(21) Application number: 14808018.7
(22) Date of filing: 02.06.2014
(51) Int. Cl.: A61K 9/28

(54) **SOLID PHARMACEUTICAL DOSAGE FORM FOR RELEASE OF AT LEAST TWO ACTIVE PHARMACEUTICAL INGREDIENTS IN THE ORAL CAVITY**
FESTE PHARMAZEUTISCHE DARREICHUNGSFORM ZUR FREISETZUNG VON WENIGSTENS ZWEI PHARMAZEUTISCHEN WIRKSTOFFEN IN DER MUNDHÖHLE
FORME DOSIFIÉE PHARMACEUTIQUE SOLIDE DE LIBÉRATION D'AU MOINS DEUX PRINCIPES PHARMACEUTIQUES ACTIFS DANS LA CAVITÉ BUCCALE

(30) Priority: 03.06.2013 SE 1300404
(43) Date of publication of application: 13.04.2016
(73) Proprietor: McNeil AB, 251 09 Helsingborg (SE)
(72) Inventor: HUGERTH, Andreas, 237 33 Bjärred (SE); LINDELL, Katarina, 241 93 Eslöv (SE); NICKLASSON, Fredrik, 237 31 Bjärred (SE); THYRESSON, Kristina, 226 57 Lund (SE)
(74) Representative: Carpmaels & Ransford LLP
(86) International application number: PCT/SE2014/050670
(87) International publication number: WO 2014/196916

(56) References cited:
- EP-A1- 1 219 291
- WO-A1-2005/013944
- WO-A1-2013/103318
- WO-A2-2005/063203
- WO-A2-2006/124366
- WO-A2-2008/008801
- WO-A2-2009/078037
- WO-A2-2010/046933
- WO-A2-2011/030351
- WO-A2-2012/085043
- WO-A2-2012/158030
- US-A- 5 098 715
- US-A1- 2007 104 783

## Description

### Technical field

The present invention relates to solid pharmaceutical dosage forms intended for release of one or more active pharmaceutical ingredients (APIs) in the oral cavity, such dosage forms being provided with means for reducing one or more organoleptically disturbing sensations.

### Background of the invention

Pharmaceuticals intended for oral administration are typically provided in solid form as tablets, capsules, pills, lozenges, or granules. Rapidly dissolving tablets are often employed in the administration of pharmaceuticals where it is impractical to provide a tablet for swallowing whole, for instance with paediatric patients. Several workers in the field have explored rapidly disintegrative tablets, e g U.S. Patent Nos. 6,106,861 and 6,024,981 and PCT Application No. WO 99/47126.

Pharmaceutical tablets for intraoral delivery of nicotine presently available on the market include Commit^{®} Lozenge or NiQuitin^{®} lozenge, a nicotine-containing tablet manufactured by GlaxoSmithKline, and Nicorette Microtab^{®} Sublingual Tablet, a nicotine-containing tablet manufactured by McNeil AB. Many subjects using said tablets experience organoleptically disturbing sensations induced by the nicotine and/or by excipients.

Hence, although release of APIs in the oral cavity and/or within the pharynx from solid pharmaceutical dosage forms is a convenient means for administration, sufficient reduction of organoleptically disturbing sensations induced by the APIs and/or by non-active Excipients of the dosage forms remains an unsolved problem.

### Prior art and problems thereof

Ingredients in pharmaceutical tablets for intraoral delivery of APIs, which seemingly could have an effect on reducing organoleptically disturbing sensations, comprise one or more flavoring agents and one or more sweeteners. Hence said one or more flavoring agents and said one or more sweeteners do not sufficiently contribute to reducing the organoleptically disturbing sensations related to intraoral delivery from the tablet. One possible reason to why the one or more flavoring agents and the one or more sweeteners do not sufficiently contribute in reducing said organoleptically disturbing sensations may be that the API has to be dissolved in the saliva in order to be absorbed. Once the API is dissolved in saliva the organoleptically disturbing sensations induced by the API cannot be sufficiently reduced. The same applies for excipients inducing organoleptically disturbing sensations.

The article "Taste Masking of Ondansetron Hydrochloride by Polymer Carrier System and Formulation of Rapid-Disintegrating Tablets, by Shagufta Khan, Prashant Kataria, Premchand Nakhat, and Pramod Yeole, published June 22, 2007 in AAPS PharmSciTech, discloses taste-masking of the bitter taste of the antiemetic drug ondansetron HCL and subsequent formulation of a rapid-disintegrating tablet (RDT) of the taste-masked drug. Such taste-masking, often called microencapsulation, is though unsatisfactory in the present context. This is because the granules are not intended to release the API in the oral cavity upon being disintegrated from the tablet in the mouth. Hence, coating of individual particles or granules according to the above article does not solve the present problem. In order to be effective NRT product nicotine has to be absorbed primarily by the oral mucosa if orally administered.

The article "Development and evaluation of paracetamol taste masked orally disintegrating tablets using polymer coating technique", International Journal of Pharmacy and Pharmaceutical Sciences, ISSN- 0975-1491 Vol 4, Suppl 3, 2012, relates to taste-masking.

The purpose of the research disclosed in the above article was to mask the intensely bitter taste of paracetamol and to formulate the orally disintegrating tablets (ODTs). Taste-Masked orally disintegrating tablets of paracetamol were prepared by Flash Tab Technology. Taste masked granules of paracetamol were prepared by coating the granules of the drug using a pH-sensitive polymer Eudragit EPO in a fluidized bed coater and the coated granules were evaluated for various parameters like Bulk density, Tapped density, Compressibility index, Hausner's Ratio and Angle of Repose. Wet granulation technique was used for the preparation of the tablets using crospovidone and hydroxypropyl cellulose as disintegrants. The tablets were evaluated for post compaction parameters like thickness, friability, weight variation etc. Disintegration time of the tablets was found to be 27 sec and almost 100% drug released in 30 minutes. The taste of the formulation was found to be acceptable by analyzing the responses of the healthy human volunteers. Thus, taste-masked orally disintegrating tablets of paracetamol can be effectively prepared by a convenient wet granulation method.

The study conclusively demonstrated complete taste masking of paracetamol and rapid disintegration and dissolution of Orally Disintegrating Tablets of Paracetamol. Coating with pH sensitive polymers Eudragit EPO and Hydroxy Methyl Cellulose effectively masked the bitter taste of paracetamol. Complete taste masking was achieved and stable mouth dissolving tablets of Paracetamol were formulated with superior organoleptic properties, excellent in vitro dispersion time and drug release almost identical to marketed preparations of Paracetamol. However, in the view of the potential utility of the formulation, stability studies were carried out at recently changed ICH conditions

The tobacco industry knows that menthol overrides the harsh taste of tobacco during smoking and alleviates nicotine's irritating effects, synergistically interacts with nicotine, stimulates the trigeminal nerve to elicit a 'liking' response for a tobacco product, and makes low tar, low nicotine tobacco products more acceptable to smokers than corresponding non-mentholated tobacco products. See "Menthol's potential effects on nicotine dependence: a tobacco industry perspective", Valerie B Yerger, Tobacco Control 2011; 20(Suppl 2):ii29eii36. doi:10.1136/tc.2010.041970. This publication though does not disclose any use of menthol for reducing one or more organoleptically disturbing sensations in solid pharmaceutical dosage forms that are characterized in that it is provided with at least one film coating for reduction for release of nicotine in the oral cavity.

WO2008008801 A2 (MCNEIL NUTRITIONALS LLC ET AL) discloses solid oral dosage vitamin and mineral compositions comprising a coating agent, a high intensity sweetener and an acid. From e g [0002], [0012] and Example 4, [00118], it is clear that the composition is for swallowing, i e peroral administration. This is contrary to the present invention, which is a solid pharmaceutical dosage form for oral administration, i e not for swallowing, but for uptake in the oral cavity.

WO2005013944 A1(MERCK FROSST CANADA INC ET AL) discloses a flavored taste-masked pharmaceutical formulation for swallowing comprising etoricoxib in a plurality of cores made using a one-step coating process. This is contrary to the present invention, which is a solid pharmaceutical dosage form for oral administration, i e not for swallowing, but for uptake in the oral cavity. Further, the present invention pertains to unitary formulations.

WO2009078034 A2 (RUBICON RES PRIVATE LTD ET AL) discloses orally disintegrating ropinorole-containing and taste-masked tablet compositions.

WO2011030351 A2 (RUBICON RES PRIVATE LTD ET AL) discloses orally disintegrating PDE-5 inhibitor-containing and taste-masked tablet compositions.

WO2010046933 A2 (RUBICON RES PRIVATE LTD ET AL) discloses orally disintegrating linezolid-containing tablet compositions being taste-masked using water-insoluble excipients.

WO2005063203 A2 (VECTURA LTD ET AL) discloses freeflowing multiparticulate formulations for oral delivery. This is in contrast to the present dosage form, which is a unitary formulation.

EP121921 A1 (MCNEIL PPC INC) discloses texture masked particles comprising an active ingredient, a film-forming polymer and an anti-grit agent. Formulations according to the present invention do not comprise any anti-grit agent.

WO200624366 A2 (WARNER LAMBERT CO ET AL) discloses flavoring of drug-containing chewing gums. The present invention does not encompass chewing gums.

US2007104783 A1 (DOMB ABRAHAM J ET AL) discloses bioadhesive sticker tablets that are to remain adherent to ulcers or lesions in the oral cavity for at least 60 minutes. Formulations according to the present invention may remain adherent to the oral cavity for less than 60 minutes.

WO2012085043 A2 (KOLTER KARL ET AL) relates to a rapidly disintegrating solid coated dosage form, with at least one film coating comprising completely or partially hydrolyzed, rapidly water-soluble polyether-vinyl ester graft polymers. WO2012158030 A2 (EMOTIONAL BRAIN B.V) relates to a dual drug delivery device, comprising a time controlled, immediate release drug delivery system comprising a first active ingredient and a second coating comprising a second active ingredient, for oral administration of the active ingredients to a subject in need thereof.

Hence, there is a need for a convenient and more efficient way to further reduce said organoleptically disturbing sensations.

### Definitions

The below definitions apply *mutatis mutandis* on expressions being similar to those being defined below.

The term "Active Pharmaceutical Ingredient (API)", also called Drug Substance, is herein intended to mean a substance or mixture of substances intended to be used in the manufacture of a drug (medicinal) product and that, when used in the production of a drug, becomes an active ingredient of the drug product. Such substances are intended to provide pharmacological activity or other direct effect in the diagnosis, cure, mitigation, treatment, or prevention of disease or to affect the structure and function of the body.

The term "intraoral" is herein intended to mean within the oral cavity.

The term "release" as a verb is herein intended to mean to liberate an API from its dosage form and to make the API available in dissolved form for subsequent absorption. The term "release" as a noun is to be understood correspondingly.

The term "organoleptically disturbing sensation" is herein intended to mean a sensation perceived as negative in the oral cavity. Non-limiting examples of such sensations are irritation, acridity, taste alteration and taste blocking, feelings of burning, astringing, bitterness and tingling, off tastes such as sour, salty, metallic, soapy, musty, sulphurous, pungent, fatty and foul tastes. Said organoleptically disturbing sensations may be induced by an API, or by non-active excipients. Non-limiting examples of such sensations specifically induced by nicotine are irritation, acridity, feelings of burning, bitterness and tingling, off tastes such as sour, salty, metallic, soapy, fatty and foul tastes. The present application encompasses organoleptically disturbing sensations regardless of their perceived intensity.

The term "organoleptically disturbing substance" is herein intended to mean a substance that may induce an organoleptically disturbing sensation. Organoleptically disturbing substances may encompass APIs, and non-active excipients. Whether a substance induces an organoleptically disturbing sensation or not may be established by methods known in the art, such as commonly used methods for characterizing organoleptic parameters of food and beverages, such as wine. Non-limiting examples of such methods are e g found in "Sensory Evaluation A practical Handbook", Sarah E. Kemp, Tracey Hollowood and Joanne Hort, Wiley-Blackwell 2011, "Sensory Evaluation Techniques, Fourth Edition, Morten C. Meilgaard, Gail Vance Civille and B. Thomas Carr, CRC Press 2007, and "Sensory Evaluation of Food, Principles and Practices, Second Edition", Harry T. Lawless and Hildegarde Heymann, Springer 2010.

The term "off taste" is herein intended to mean an unpleasant taste or an unpleasant after taste.

The term "coat" is herein intended to mean cover entirely or partly

The term "core" is herein intended to mean an uncoated solid pharmaceutical dosage form. In other words a core is what you place a coating on to get a coated solid pharmaceutical dosage form. One may also say that a core is encapsulated with a coating to get a coated solid pharmaceutical dosage form.

### Summary of the invention

The present invention seeks to address the problem of needing to reduce one or more organoleptically disturbing sensations induced by one or more organoleptically disturbing substances being released in the oral cavity from an API-containing solid dosage form.

The invention provides a solid pharmaceutical dosage form for oral administration comprising:
(a) a core comprising at least one active pharmaceutical ingredient (API) selected from the group consisting of anesthetics, antibiotics, anti-inflammatory drugs, anti-migraine drugs, antiseptics, anti-diarrhea drugs, decongestants, erectile dysfunction drugs, mucolytics, muscle relaxants, anti-allergy drugs, stimulants, substances for treatment of oral malodour, antihistamines, expectorants and cough suppressants, wherein the core of the solid dosage form has a weight from 50 mg to 2000 mg, and
(b) a film coating having a weight of from 1% to 15% of the weight of the core, comprising at least one film-forming polymer wherein the film-forming polymer is hydroxypropyl methylcellulose, at least one flavoring agent, and at least one sweetener, wherein the film coating is devoid of any API, wherein the solid pharmaceutical dosage form comprises at least two APIs,
wherein the solid pharmaceutical dosage form optionally comprises one or more inactive component, wherein said solid pharmaceutical dosage form is selected from the group consisting of a lozenge, a sublingual tablet, a buccal tablet and an orally disintegrating tablet, whereby upon administration the API is capable of being completely dissolved in the oral cavity wherein one or more organoleptically disturbing sensations induced by one or more of the APIs and/or of inactive components of the solid pharmaceutical dosage form is/are reduced by constituents of said film coating, and wherein said dosage form does not contain nicotine.

Thus, the invention provides a solid pharmaceutical dosage form for oral administration comprising a core coated by at least one film coating, where the dosage form comprises at least one Active Pharmaceutical Ingredient (API), which is not nicotine, for release in the oral cavity. The perception of one or more organoleptically disturbing sensations induced by the dosage form is reduced by constituents of said film coating, said constituents comprising at least one film-forming polymer and at least one flavorant or at least one sweetener. Preferably said constituents comprise at least one film-forming polymer, at least one flavorant and at least one sweetener.

Preferably, the reduction of the one or more organoleptically disturbing sensations is the result of synergism between said constituents. This may also be expressed as the result of a synergistic action and/or interaction between said constituents.

The present invention encompasses reduction of one or more organoleptically disturbing sensations whatever the mechanism or mechanisms behind said reduction.

The reduction may e g be a result of the interacting constituents reducing the subject's sensitivity for such sensations. The reduction may also be a result of the interacting constituents reducing the induction of one or more organoleptically disturbing sensations. A combination of the foregoing mechanisms is possible. Other mechanisms are also envisagable.

Optionally the dosage form may comprise a further API, e g zinc acetate and other salts or complexes with zinc.

Said reduction of organoleptically disturbing sensations preferably does not noticeably deteriorate the pharmaceutical effect of the API(s).

### Detailed description of the invention

The present solid pharmaceutical dosage form mainly erodes in the mouth whereby the APIs are released and exposed to intraoral sensory receptors, e g taste receptors and trigeminal receptors. Preferably the APIs are essentially absorbed by the mucosa of the oral cavity. Examples of said pharmaceutical dosage form are tablet dosage forms intended to be completely dissolved in the oral cavity, and are selected from the group consisting of lozenges, sublingual tablets, buccal tablets and orally disintegrating tablets. Said solid pharmaceutical dosage form is not intended to be swallowed.

The present problem is also of specific interest for certain excipients, non-limiting examples of which are buffers, such as carbonate (including bicarbonate or sesquicarbonate), glycinate, different phosphate systems such as trisodium phosphate, disodium hydrogen phosphate; and tripotassium phosphate, dipotassium hydrogen phosphate, glycerophosphate or citrate of an alkali metal (such as potassium or sodium, or ammonium), e g trisodium and tripotassium citrate, different hydroxides, amino acids, and mixtures thereof, and other excipients that may induce organoleptically disturbing sensations.

When you administer an API, with a solid pharmaceutical dosage form the API is continuously released as long as the dosage form remains in the mouth. If you do not suck or otherwise mechanically process the dosage form, less API, and less excipients, is released compared to if you suck and/or otherwise mechanically process it. By stopping to suck and/or otherwise mechanically process the dosage form said organoleptically disturbing sensations are normally still not sufficiently reduced.

One way to sufficiently reduce said organoleptically disturbing sensations for a lozenge or a sublingual tablet could be to remove the dosage form from the mouth and put it back into the mouth once the organoleptically disturbing sensations have sufficiently waned. This is though a very inconvenient way to reduce said organoleptically disturbing sensations. For fast dissolving tablets and rapidly disintegrating tablets this option is not available as these tablets would fall apart if they should be taken out from the mouth.

The intention with the present invention is to keep the dosage form in the oral cavity until substantially dissolved or disintegrated and still reduce organoleptically disturbing sensations. If the dosage form instead would be temporarily removed from the mouth as described above this would be not only very inconvenient, but the release of the API would be temporarily stopped, which normally is unwanted *inter alia* because that may affect the intended dosage regime.

Pharmaceutical tablets for intraoral delivery of nicotine presently available on the market include Commit^{®} Lozenge or NiQuitin^{®} lozenge, a nicotine-containing tablet manufactured by GlaxoSmithKline, and Nicorette Microtab^{®} Sublingual Tablet, a nicotine-containing tablet manufactured by McNeil AB. Many subjects using said tablets experience organoleptically disturbing sensations induced by nicotine.

Ingredients in said tablets, which seemingly could have an effect on reducing organoleptically disturbing sensations, comprise one or more flavoring agents and one or more sweeteners. Hence said one or more flavoring agents and said one or more sweeteners do not sufficiently contribute to reducing the organoleptically disturbing sensations related to intraoral delivery from the tablet. One reason to why the one or more flavoring agents and the one or more sweeteners do not sufficiently contribute in reducing said organoleptically disturbing sensations may be that the API has to be dissolved in the saliva in order to be absorbed. Once the API is dissolved in the oral cavity the organoleptically disturbing sensations induced by the API cannot be reduced. The same applies for excipients inducing organoleptically disturbing sensations.

The experience is similar for other APIs released in the oral cavity. An API usually has several clinical indications. Examples of clinical indications and APIs are
a) anesthetics e.g. benzocaine, cocaine, dyclonine, lidocaine;
b) antibiotics e.g. amphotericin, chlorotetracycline, domiphen bromide, doxycycline, gramicidin, minocycline, natamycin, neomycin, tetracycline, tyrothricin;
c) anti-inflammatory drugs e.g. acetyl salicylic acid, amlexanox, becaplermin, benzydamine, dexamethasone, flurbiprofen, ibuprofen, paracetamol;
d) anti-migraine drugs e.g. triamcinolone, rizatriptan, sumatriptan, zolmitriptan;
e) antiseptics e.g. ambazone, benzoxonium chloride, cetrimonium chloride, cetylpyridinium chloride, chlorhexidine, clotrimazole, hexamidine, hexetidine, hexylresorcinol, metronidazole, miconazol, oxyquinoline, tibezonium iodide;
f) decongestants e.g. phenylephrine;
g) anti-diarrhea drugs e.g. loperamide, racecadotril;
h) erectile dysfunction drugs e.g. sildenafil;
i) mucolytics e.g. acetylcysteine, ambroxol, bromhexin, carbocysteine, domiodol, eprazinon, erdosteine, etosteine, sobrerol, stepronin;
j) muscle relaxants e.g. methocarbamol;
k) anti-allergy drugs e.g. diphenhydramine;
l) stimulants e.g. caffeine;
m) substances for treatment of oral malodour e.g. zinc salts.
n) antihistamines e.g. meclozine, chlorphenamine maleate, pheniramine maleate, ceterizine;
o) expectorants e.g. guaifenesin, guaietolin, and
p) cough suppressants e.g. dropropizine, dextromethorphan

One or more of the API(s) may be in non-coated particulate form.

The present invention provides a solution to the above-mentioned problem of reducing one or more organoleptically disturbing sensations induced by one or several of the APIs and/or of inactive components of the solid pharmaceutical dosage form. The solution resides in providing said solid dosage form with at least one film coating, the constituents of which comprise at least one film-forming polymer and at least one flavorant and at least one sweetener.

Said at least one film coating is devoid of any other API and preferably devoid of buffer.

Said reduction in perception of organoleptically disturbing sensations preferably does not significantly affect the release of the API.

The core of the present solid dosage form has a weight from 50 mg to 2000 mg, more preferably from 90 mg to 1200 mg. The film coating on the core has a weight of from 1 % to 15 % of the weight of the core.

The thickness of the film coating has an influence on the degree of reduction of the organoleptically disturbing sensations. Preferably the film coating has an average thickness from 10 to 500 microns, more preferably from 20 to 250 microns, and most preferably from 30 to 150 microns. The actual film thickness is adapted in dependence of different parameters, such as the organoleptic sensation to be reduced, the concentration of flavour, the type of flavour sweetness compounds used and their relative levels and amounts used. The film thickness may be measured using different methods known in the art such as SEM (Scanning Electron Microscopy), digital micrometer, X-ray microtomography, terahertz pulsed imaging etc. See further e g Quantitative Analysis of Film Coating in a Pan Coater Based on In-Line Sensor Measurements, Jose D. Perez-Ramos et al, AAPS PharmSciTech 2005; 6 (1) Article 20, Nondestructive analysis of tablet coating thicknesses using terahertz pulsed imaging. J Pharm Sci. 2005; 94:177Y183. Fitzgerald AJ, Cole BE, Taday PF., Hancock B, Mullarney MP. X-ray microtomography of solid dosage forms. Pharm Technol. 2005;29:92Y100.

A rapid dissolution or disintegration of the at least one film coating is instrumental for not impairing the release of the APII. Hence, it is of importance that to an essential degree the at least one film coating dissolves or disintegrates rapidly, preferably in less than 2 minutes, more preferably in less than 1 minute and most preferably in less than 30 seconds, from the moment of administration

Too long a time for release of the API may impair the user friendliness. Hence, the solid dosage form may preferably release the API within 30 minutes, more preferably within 15 minutes, from the moment of administration.

The film coating comprises at least one film-forming polymer wherein the film-forming polymer is hydroxypropyl methylcellulose. Film-forming polymers include cellulose ethers e g hydroxy propyl methyl cellulose (HPMC), methyl hydroxy ethyl cellulose (MHEC), hydroxy propyl cellulose (HPC), hydroxyethyl cellulose (HEC), ethyl hydroxyl ethyl cellulose (EHEC), and other film forming polymers such as methacrylic acid copolymer-type C sodium carboxy methyl cellulose, polydextrose, polyethylene glycols, acrylate polymers (e g poly vinyl acrylate (PVA)),polyvinyl alcohol-polyethylene glycol graft copolymers, complex of polyvinylpyrrolidone (PVP), such as povidone, polyvinyl alcohol, microcrystalline cellulose, carrageenan, pregelatinized starch, polyethylene glycol, and combinations thereof. Typically, the molecular weight (weight average and/or number average) of the polymer is from 1,000 to 10,000,000, preferably from 10,000 to 1,000,000, as measured by e.g. gel permeation chromatography.

Optionally, a plasticizer may be added to the film-forming polymer to facilitate the spreading and film forming capability. Examples on useful plasticizers are glycerol, propylene glycol, polyethylene glycol (PEG 200-6000), organic esters e g triacetin (glyceryl triacetate), triethyl citrate, diethyl phtalate, dibutyl phtalate, dibutyl sebacete, acetyltriethyl citrate, acethyltributyl citrate, tributyl citrate, and oils/glycerides such as fractionated coconut oil, castor oil and distilled acetylated monoglycerides. Additionally, or alternatively, surfactants may be included to facilitate the incorporation of flavors and to improve penetration and spreading properties of the coating liquid. Non-limiting examples of surfactant are polysorbates derived from PEG-ylated sorbitan esterified with fatty acids such as Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 80 (Polyoxyethylene (20) sorbitan monooleate) (e g Tween 80, Tween 40, Tween 20), sodium lauryl sulphate (SLS), poloxamer surfactants i.e. surfactants based on ethylene oxide - propylene oxide block copolymers and other surfactants with high HLB-value.

Anti-tacking agents/glidants may in a non-limiting way be chosen among compounds such as talc, magnesium stearate, kaolin, colloidal silicon dioxide and glyceryl monostearate. The aforementioned agents may also be included to reduce sticking issues.

The flavoring agents may in a non-limiting way be chosen among natural or synthetic flavouring or aromatizing agents and may be added as liquids and/or as powder. Flavour and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavour of the fruit, (e g strawberry, raspberry, black currant, banana, melon, cherry, passion fruit, pineapple, peach, blackberry, mango, papaya, guava, cranberry, cloudberry, violet, pomegranate, pear, apple); artificial and natural flavours of brews and liquors, (e g cognac, whisky, rum, gin, sherry, port, and wine); tobacco, coffee, tea, cocoa, and mint; fruit juices including expelled juice from washed, scrubbed fruits such as lemon, orange, lime and other citric fruits; spear mint, pepper mint, lemon balm, wintergreen, cinnamon, cacoe/cocoa, vanilla, liquorice, menthol, eucalyptus, aniseeds, nuts (e g peanuts, coconuts, hazelnuts, chestnuts, walnuts, colanuts), almonds, raisins and ginger; and powder and flour.

The sweeteners may in a non-limiting way be chosen among synthetic or natural sugars, i e any form of carbohydrates suitable for use as sweetener, as well as so called artificial sweeteners such as saccharin, sodium saccharin, aspartame, e g NutraSweet^{®}, acesulfame or Acesulfame K^{®}, potassium acesulfame, thaumatin, glycyrrhizin, sucralose, dihydrochalcone, miraculin, monellin, stevside, e g Stevia^{®}, neotame, N-substituted APM derivatives, cyclamic acid and its salts and alitame. Sweeteners may also be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, (e g starch hydrolysates, containing a mixture of dextrose, maltose and a range of complex sugars), invert sugar syrup, (e g sucrose inverted by invertase (also called sucrase or sacchrase) containing a mixture of dextrose and fructose), high sugar content syrups such as treacle and honey containing a mixture of particular leavulose, dextrose, maltose, lactitole, sucrose, resins, dextrin and higher sugars; and malt or malt extracts.

Preferably the coating is devoid of added acids.

Other adjuvants may also be included in the composition of the film such as coloring agents, opacifiers, glossing agents, pore forming agents, excipient stabilizers.

The dosage form is devoid of nicotine.

The dosage form is preferably devoid of etoricoxib, ropinirole, PDE-5 inhibitors and linezolid.

The dosage form is preferably devoid of anti-grit agents.

The dosage form preferably has a bioadherence being such that it may remain adhered to the mucosa of the oral cavity for a maximum of 60 minutes.

The dosage forms of the invention may be prepared by way of a variety of routine techniques, and using standard equipment, known to the skilled person (see, for example, Lachman et al, "The Theory and Practice of Industrial Pharmacy", Lea & Febiger, 3rd edition (1986) and "Remington: The Science and Practice of Pharmacy", Gennaro (ed.), Philadelphia College of Pharmacy & Sciences, 19th edition (1995)). For example, a core comprising nicotine is first produced using known tabletting techniques, which is then coated with a solution containing a film-forming polymer.

Standard mixing equipment may be used for mixing together components of compositions of the invention. The mixing time period is likely to vary according to the equipment used, and the skilled person will have no difficulty in determining by routine experimentation a suitable mixing time for a given combination of ingredient(s).

Surprisingly, after that the film coating essentially has disappeared from the surface of the solid dosage form the reduction of organoleptically disturbing sensations remains.

Equally surprisingly, when incorporating said components for reducing organoleptically disturbing sensations in the core of the solid dosage form, instead of incorporating those in the film coating said organoleptically disturbing sensations will not be sufficiently reduced or reduced to the same extent.

Upon it having been dissolved a film coating on its own has a limited effect on the reduction of organoleptically disturbing sensations. A component for reduction of said sensations, such as a flavoring agent or a sweetener, may have a limited effect on its own on the reduction of organoleptically disturbing sensations. Surprisingly the combined effect of a film coating and at least one further component for reduction of said sensations, provides an effect that is more profound than the sum of the effects of the film coating on its own and the at least one further component on its own.

Reducing organoleptically disturbing sensations implies increased therapy adherence, which may lead to increased efficacy of the treatment.

### Examples

The below examples on embodiments and manufacturing of the present formulations as well as on testing the present formulations are non-limiting and for illustrating the present invention. Examples are given for some of the APIs and the clinical indications are not explicitly stated in the examples since an API may have several clinical indications and their clinical indications may be changed e.g. new indications may be discovered and ascribed to the APIs. Alternatives and variations of the below examples within the scope of the present invention as per the below claims may be carried out by a person skilled in the art. Ingredients as per the below examples may be exchanged for equivalent ingredients. The combination of tablet cores and film coatings in the examples given are arbitrary. Any film coating can be combined with any tablet core.

### Reference Example 1: Diphenhydramine Lozenge

### Manufacturing method

The composition for a batch of tablet cores is given below in Table A1. The materials are sieved using an oscillating sieve with 1mm mesh size and thereafter blended, according to methods known in the art e g using a double cone blender, for sufficient time (e.g. 10 to 30 minutes) to reach an acceptable blending homogeneity of the API(s) i e RSD ≤5%. The blended materials are then compressed into tablets by means of direct compression. The powder compression may for example be performed using a rotary tablet press with concave punches. The tablets are compressed to sufficient hardness to have a friability of ≤ 1 % to withstand shear forces in the coating process and to achieve the desired *in vivo* dissolution time.

**Table 1A: Components of the tablet core.**

| Ingredients | Percent (w/w) | mg/portion |
|---|---|---|
| Diphenhydramine hydrochloride* | 0.59 | 5 |
| Ammonium chloride* | 5.88 | 50 |
| Sodium citrate* | 1.18 | 10 |
| Mannitol | 85.12 | 723.5 |
| Xanthan gum | 1.76 | 15 |
| Lime-Honey Flavor | 1.76 | 15 |
| Vanilla Flavor | 1.18 | 10 |
| Sucralose | 0.18 | 1.5 |
| Magnesium stearate | 2.35 | 20 |
| TOTAL | 100 | 850 |

Table 1B provides numerous alternative non-limiting examples of tablet core compositions.

**Table 1B: Components of the tablet core.**

| Ingredients | Percent (w/w) | mg/portion |
|---|---|---|
| Diphenhydramine hydrochloride* | 0.27 - 5.38 | 2.5 - 50 |
| Ammonium chloride | 0-5.38 | 0-50 |
| Sodium citrate | 0 - 1.08 | 0-10 |
| Polyol (preferably directly compressible (DC) grade)** | 0 79.3 - 99.11 | 737.5 - 921.75 |
| Xanthan gum*** | 0-3.23 | 0-30 |
| Flavor (e g Lemon/lime/eucalyptus/mint/vanilla/forest fruit) | 0.054 - 2.69 | 0.5 - 25 |
| Sucralose **** | 0.027 - 0.27 | 0.25 - 2.5 |
| Magnesium stearate | 0.54-2.69 | 5-25 |
| TOTAL | 100.0 | 930.0 |

| | | |
|---|---|---|
| * or other source equivalent to the interval diphenhydramine hydrochloride given. ** or other fillers e.g. lactose(DC), sucrose *** or other gelling agent **** or other high intensity sweetener or combination of such sweeteners. | | |

Film coating of the tablets can be performed using e g a standard modern pan coater equipped inter alia with air atomized spray nozzles to distribute the film coating fluid and a perforated drum of appropriate size. The film solution is prepared by adding the hydroxypropyl methylcellulose and plasticizer (if such is included in the composition) to purified water (>85°C) whilst stirring. The most suitable temperature of the solvent used for dispersing the hydroxypropyl methylcellulose depends on the type of hydroxypropyl methylcellulose used. There is abundant information in the literature regarding hydroxypropyl methylcellulose film preparation e g from polymer manufacturers such as Dow Inc. http://dowwolff.custhelp.com/app/answers/detail/a_id/1094/kw/prepare/session/L3RpbW UvMTMyMzY3MzM3Ny9zaWQvMkFoOUVuTGs%3D and http://dowwolff.custhelp.com/app/answers/detail/a_id/1181. The film solution is cooled to approximately 20°C and sucralose is added when the solution is approximately 40°C. The solution is allowed to settle at ambient conditions for at least 3 hours where after the solution is homogenized using a Silverson homogenisator. Thereafter flavor mixture is added containing e.g. Polyoxyethylene (80) sorbitan monooleate and flavor e.g. mint.

The resulting mixture is stirred until it is homogenous. The components of the film coating composition are given below and in other examples are provided as the calculated amount per unit dosage form. The sum of the "dry excipients", also referred to as "solids content" is usually in the range 5-25 % w/v of the total coating solution. The actual solids content chosen depends also on the composition, coating process parameters and coating equipment.

**Table 1C: Components of the film coating.**

| Ingredients | Percent (w/w) | mg/portion |
|---|---|---|
| Hydroxypropyl methylcellulose | 79.7 | 19.925 |
| Polyoxyethylene (20) sorbitan monooleate | 0.3 | 0.075 |
| Sucralose* | 8.0 | 2 |
| Flavor (e g Lemon/lime/eucalyptus/mint/vanilla/forest fruit) | 12.0 | 3 |
| Sum "Dry" Excipients | 100.0 | 25 |
| Aqua pur** | q.s. | - |

| | | |
|---|---|---|
| * or other high intensity sweetener or combination of such sweeteners. ** Aqua. Pur. is added q.s. to achieve a dry content suitable for the coating process parameter setting to be applied e.g. dry content in the range 10% w/w to 25% w/w. | | |

A coloring component may also be included, e g titanium dioxide.

**Table 1D: Components of an alternative film coating**

| Ingredients | Percent (w/w) | mg/portion |
|---|---|---|
| Hydroxypropyl methylcellulose | 80.45 | 32.28 |
| Polyethyleneglycol 400* | 8.2 | 3.28 |
| Polyoxyethylene (20) sorbitan monooleate | 0.1 | 0.04 |
| Titanium dioxide | 6.0 | 2.4 |
| Aspartame | 4.0 | 1.6 |
| Flavor (e g lemon/lime/eucalyptus/mint/vanilla/forest fruit) | 1.25 | 0.5 |
| Sum "Dry" Excipients | 100.0 | 40 |
| Aqua pur* | q.s. | - |

| | | |
|---|---|---|
| * Aqua. Pur. is added q.s. to achieve a dry content suitable for the coating process parameter setting to be applied. | | |

**Table 1E: Components of additionally non-limiting alternative film coatings**

| Ingredients | Percent (w/w) | mg/portion |
|---|---|---|
| Hydroxypropyl methylcellulose¹ | 44.5-97.0 | 8.9-19.4 |
| Polyethyleneglycol 400² | 0-25 | 0-5 |
| Polyoxyethylene (20) sorbitan monooleate³ | 0-0.5 | 0-0.1 |
| Titanium dioxide (optional ingredient) | 0-10 | 0-2 |
| Sucralose⁴ | 0.5-10 | 0.1-2 |
| Fruit flavor (or e g a Mint flavor)⁵ | 2.5-10 | 0.5-2 |
| Sum "Dry" Excipients | 100.0 | 20 |
| Aqua pur⁶ | q.s. | - |

| | | |
|---|---|---|
| ¹ The hydroxypropyl methylcellulose may e g be of type methocel E3, K4, E5 or F_VLV. The hydroxypropyl methylcellulose may also be replaced in part or in its entire by a combination of other film forming polymers. ² May be exchanged for propylene glycol, glycerol triacetin or other plasticizer. ³ May be exchanged for other surfactant. ⁴ Alternatively sodium lauryl sulphate or equivalent surfactant. ⁵ Alternatively other high intensity sweetener or combination of such sweeteners. Sweetener may also be included in the flavor. ⁶ Aqua. Pur. is added q.s. to achieve a dry content suitable for the coating process parameter setting to be applied and is essentially evaporated during the process. | | |

### Example 2: Benzocaine lozenge

Manufacturing method of tablets as per Example 1

**Table 2A: Components of the tablet core.**

| Ingredients* | Percent (w/w) | mg/portion |
|---|---|---|
| Benzocaine*** | 0.25 | 1.5 |
| Chlorhexidine dihydrochloride*** | 0.83 | 5 |
| Isomalt**** | 92.25 | 553.48 |
| Flavor****** (e g lemon/lime/eucalyptus/mint/vanilla/forest fruit/herbs) | 1.67 | 10.02 |
| Xanthan gum****** | 2.50 | 15 |
| Magnesium stearate****** | 2.00 | 12 |
| Silicon dioxide (colloidal) ****** | 0.50 | 3 |
| TOTAL | 100 | 600 |

| | | |
|---|---|---|
| * Benzocaine dose may e.g. be in the interval 1-3 mg with accordingly adjusted amount of polyol. ** This combination of APIs may be exchanged for other APIs such as but not limited to Ambroxol 30 mg, Dextromethorphan 7.5 mg or flurbiprofen 8,75 mg, gramicidin 0.3 mg, cetylpyridinium chloride 2 mg, amyl metacresol 0.6 mg and/or 2,4-dichlorobenzyl alcohol 2 mg, where the two latter preferably are co-administered with an acid e.g. tartaric acid and the amount of filler, Mannitol, is adjusted accordingly. The given levels of API are examples. *** If not included then adjust filler level. **** Isomalt may exchanged by other DC grade polyol e.g. mannitol, other equivalent filler. ****** The amounts may be optimized for each API-filler combination. | | |

Film coating of the tablets produced in 2A can be performed using the composition listed in Table 2B or 1C-1E and the manufacturing process can be performed using e g a standard modern pan coater equipped with air atomized spray nozzles to distribute the film coating fluid and a perforated drum of appropriate size. The film solution is prepared by adding the hydroxypropyl methylcellulose to aqua purificata during stirring and then the solution is allowed to settle overnight at ambient conditions where after polyvinyl alcohol, polyethylene glycol 400 and sucralose are added during stirring. The solution is homogenized using a Silverson homogenisator. Thereafter flavor mixture containing Polyoxyethylene (80) sorbitan monooleate and mint flavor is added. The resulting mixture is stirred until it is homogenous.

**Table 2B: Components of the film coating.**

| Ingredients | Percent(w/w) | mg/portion |
|---|---|---|
| Hydroxypropyl methylcellulose | 56.5 | 14.13 |
| Polyvinyl alcohol | 12.0 | 3.0 |
| Polyethyleneglycol 400* | 16.0 | 4.0 |
| Polyoxyethylene (80) sorbitan monooleate | 0.3 | 0.08 |
| Sucralose | 7.2 | 1.8 |
| Mint flavor | 8 | 2 |
| Sum "Dry" Exipients** | 100 | 25 |
| Aqua pur*** | q.s. | - |

| | | |
|---|---|---|
| * Or other plasticizer e g triacetin, i.e. 1,2,3-triacetoxypropane, glycerol or propylenglycol, which usually are used at concentration of 10-35% based on polymer weight. ** Sum excipients other than Aqua pur. ***Aqua. Pur. is added q.s. to achieve a dry content suitable for the coating process parameter setting to be applied e g may the dry content be 16% w/w. | | |

### Reference Example 3a: Phenylephrine lozenge

| Ingredients | Percent (w/w) | mg/portion |
|---|---|---|
| Phenylephrine hydrochloride* | 1.67 | 10 |
| Mannitol** | 93.56 | 561.36 |
| Mint flavor | 1.67 | 10.02 |
| Cooling agent | 0.1 | 0.6 |
| Magnesium stearate | 2 | 12.0 |
| Silicon dioxide (colloidal) | 0.5 | 3.0 |
| TOTAL | 100 | 600 |

| | | |
|---|---|---|
| ** other fillers such as, lactose, sucrose preferably DC grade of lactose | | |

### Example 3b: Phenylephrine and diphenhydramine lozenge

| Ingredients | Percent (w/w) | mg/portion |
|---|---|---|
| Phenylephrine hydrochloride* | 1.67 | 10 |
| Diphenhydramine hydrochloride 25 mg | | 25 |
| Isomalt** | 93.56 | 561.36 |
| Mint flavor (or other flavor/flavor combination) | 1.67 | 10.02 |
| Cooling agent | 0.1 | 0.6 |
| Magnesium stearate | 2 | 12.0 |
| Silicon dioxide (colloidal) | 0.5 | 3.0 |
| TOTAL | 100 | 600 |

Coating of the tablets produced in 3A or 3B can be performed using the compositions and procedures described vide supra.

### Reference Example 4: Zolmitriptan lozenge

Manufacturing method as per Example 1.

**Table 4A: Components of the tablet core.**

| Ingredients | Percent (w/w) | mg/portion |
|---|---|---|
| Zolmitriptan | 0.31 | 2.5* |
| Mannitol | 94.47 | 744 |
| Mint Flavor | 1.00 | 8.0 |
| Acesulfame potassium | 0.16 | 1.25 |
| Xanthan gum | 1.56 | 12.5 |
| Magnesium stearate | 100.00 | 20.0 |
| TOTAL | 0.31 | 800.0 |

| | | |
|---|---|---|
| * Zolmitriptan dose may be 2-6 mg. then the amount of polyol is adjusted accordingly. | | |

Film coating of the tablets can be performed using e g a standard modern pan coater equipped inter alia with air atomized spray nozzles to distribute the film coating fluid and a perforated drum of appropriate size.

**Table 4B: Components of the film coating.**

| Ingredients | Percent( w/w) | mg/portion |
|---|---|---|
| Hydroxypropyl methylcellulose | 75 | 26.25 |
| Triacetin*, i e 1,2,3-triacetoxypropane | 7.5 | 2.625 |
| Polyoxyethylene (80) sorbitan monooleate | 0.1 | 0.035 |
| Sucralose | 7.4 | 2.59 |
| Mint flavor | 10 | 3.5 |
| Sum "Dry" Exipients | 100 | 35 |
| Aqua pur** | q.s. | - |

| | | |
|---|---|---|
| * May be exchanged for another plasticizer, such as polyethyleneglycol 1000. **Aqua. Pur. is added q.s. to achieve a dry content suitable for the coating process parameter setting to be applied. The concentration of hydroxypropyl methylcellulose may for example be 7% w/w. | | |

### Reference Example 5.

Manufacturing method as per Example 1.

**Table 5A: Components of the tablet core.**

| Ingredients | Percent (w/w) | mg/portion |
|---|---|---|
| Sildenafil | 8.55 | 25* |
| Xylitol | 10 | 10 |
| Crospovidone (polyvinylpyrrolidone) | 2 | 2 |
| Sodium carbonate anhydrous | 5 | 5 |
| Mint flavor | 5 | 5 |
| Magnesium stearate | 0.9 | 0.9 |
| Colloidal silicon dioxide | 0.45 | 0.45 |
| TOTAL | 100 | 100 |

| | | |
|---|---|---|
| * The dose sildenafil, which may be citrate or other sildenafil source, may be in the interval 25 mg to 100 mg. | | |

Film coating of the tablets can be performed using e g a standard modern pan coater equipped inter alia with air atomized spray nozzles to distribute the film coating fluid and a perforated drum of appropriate size.

**Table 5B: Components of the film coating.**

| Ingredients | Percent (w/w) | mg/portion |
|---|---|---|
| Hydroxypropyl methylcellulose | 74 | 7.4 |
| Polyoxyethylene (80) sorbitan monooleate | 0.1 | 0.01 |
| Sucralose | 4.9 | 0.49 |
| Acesulfame potassium (Potassium 6-methyl-2,2-dioxo-oxathiazin-4) | 4 | 0.4 |
| Aspartame ((*N*-(L-α-Aspartyl)-L-phenylalanine) | 2 | 0.2 |
| Mint flavor | 15 | 1.5 |
| Sum "Dry" Exipients | 100 | 10 |
| Aqua pur* | q.s. | - |

| | | |
|---|---|---|
| *Aqua. Pur. is added q.s. to achieve a dry content suitable for the coating process parameter setting to be applied, e g 24% w/w. | | |

### Reference Example 6

Manufacturing method as per Example 1.

Components of the tablet core as per Example 4.

Film coating of the tablets can be performed using e g a standard modern pan coater equipped inter alia with air atomized spray nozzles to distribute the film coating fluid and a perforated drum of appropriate size. The film solution is prepared by adding the hydroxypropyl methylcellulose to aqua purificata whilst stirring. The film solution is cooled to approximately 20°C and sucralose and acesulfame K is added when the solution is approximately 40°C. The solution is allowed to settle at ambient conditions for at least 3 hours where after the solution is homogenized using a Silverson homogenisator. Thereafter flavor mixture is added containing Polyoxyethylene (80) sorbitan monooleate and mint flavor. The resulting mixture is stirred until it is homogenous.

**Table 6B: Components of the film coating.**

| Ingredients | Percent (w/w) | mg/portion |
|---|---|---|
| Hydroxypropyl methylcellulose | 70 | 24.3 |
| Titan dioxide | 3 | 1.05 |
| Propylene glycol | 9 | 3.15 |
| Polyoxyethylene (80) sorbitan monooleate | 0.1 | 0.035 |
| Aspartame | 4.9 | 1.715 |
| Acesulfame Potassium | 3 | 1.05 |
| Mint flavor | 10 | 3.5 |
| Sum "Dry" Exipients | 100 | 35 |
| Aqua pur* | q.s. | - |

| | | |
|---|---|---|
| * Aqua. Pur. is added q.s. to achieve a dry content suitable for the coating process parameter setting to be applied. | | |

### Reference Example 7

As per Example 6, but with the following film coating composition:

**Table 7B: Components of the film coating.**

| Ingredients | Percent (w/w) | mg/portion |
|---|---|---|
| Hydroxypropyl methylcellulose | 77.3 | 11.595 |
| Titan dioxide | 3 | 0.45 |
| Polyethyleneglycol 400 | 1.5 | 0.225 |
| Sodium lauryl sulfate | 0.3 | 0.045 |
| Aspartame | 4.9 | 0.735 |
| Acesulfame Potassium | 3 | 0.45 |
| Mint flavor | 10 | 1.5 |
| Sum "Dry" Exipients | 100 | 15 |
| Aqua pur* | q.s. | - |

| | | |
|---|---|---|
| * Aqua. Pur. is added q.s. to achieve a dry content suitable for the coating process parameter setting to be applied. | | |

### Reference Example 8

Manufacturing method as per Example 1.

Coating as per Example 4.

**Table 8A: Components of the tablet core**

| Ingredients | Percent (w/w) | mg/portion |
|---|---|---|
| Diphenhydramine hydrochloride | 3.13 | 25.00 |
| Mannitol | 93.68 | 749.40 |
| Mint flavour | 1.25 | 10.00 |
| Cooling agent | 0.08 | 0.60 |
| Magnesium stearate | 1.88 | 15.00 |
| TOTAL | 100.00 | 800.00 |

### Reference Example 9

### Manufacturing method

The compositions for two tablet cores are given below in Table 9A. The master granule materials are sieved using an oscillating sieve with 1mm mesh size and thereafter blended, according to methods known in the art e.g. using a double cone blender for 10 to 30 minutes. The blended materials are then wetted with purified water. The wet mass is then feeded to an extruder to form the granules. The resultant granules are dried using any method known in the art, such as fluid bed drying. The master granules are then screened for a suitable particle size, typically 75 µm, and 200 mesh. The master granules are then blended with the Ambroxol active, at least one buffering agent, flavorants and sweeteners. Upon mixing and screening a lubricant or glidant is added to the mixture. The tablets are compressed to sufficient hardness to enable an acceptable coating process and to achieve the desired *in vivo* dissolution time.

**Table 9 A Components of core.**

| Ingredients | | Formulation 9A mg/portion | Formulation 9B mg/portion |
|---|---|---|---|
| | *Master granule:* | | |
| Mannitol | | 186.8 | 1062.6 |
| Sodium alginate | | 10.30 | 63.70 |
| Xanthan qum | | 1.99 | 12.25 |
| Calcium polycarbophil | | 5.13 | 31.73 |

| | *Dry mixed components:* | | |
|---|---|---|---|
| Ambroxol | | 20 | 20 |
| Aspartame | | | 6.00 |
| Acesulfame Potassium | | 1.50 | |
| Mint flavour | | 21.25 | 1.2 |
| Magnesium stearate | | 2.50 | 2.50 |
| Total weight of tablet core mg | | 250 | 1200 |

**Table 9 B: Components of film coating.**

| Ingredients | Percent (w/w) | Formulation 9 A mg/portion | Formulation 9 B mg/portion |
|---|---|---|---|
| Hydroxypropyl methylcellulose | 77.3 | 4.83 | 23.19 |
| Titan dioxide | 3 | 0.19 | 0.90 |
| Polyethyleneglygol 400 | 1.5 | 0.094 | 0.45 |
| Sodium lauryl sulfate | 0.3 | 0.019 | 0.09 |
| Aspartame | 4.9 | 0.31 | 1.47 |
| Acesulfame Potassium | 3 | 0.19 | 0.90 |
| Mint flavor | 10 | 3 | 3.00 |
| Sum "Dry" Exipients | 100 | 8.633 | 30 |
| Aqua Pur* | q.s. | - | - |

| | | | |
|---|---|---|---|
| * Aqua. Pur. is added q.s. to achieve a dry content suitable for the coating process parameter setting to be applied. | | | |

The respective amounts in the two above formulations 9 A and 9 B may vary within an interval of +- 15 % (w/w), preferably within + - 5 % (w/w) without thereby deviating from the desired characteristics for the respective formulations.

### Example 10

Results from a sensory study confirmed the surprising finding of reduction of disturbing sensations. 10 study persons (healthy volunteers; 6 males and 4 females in age range 40 to 64 years) completed the study and compared two chlorhexidine lozenge 5 mg formulations; lozenge A, uncoated, with all of flavoring agents and sweeteners in the tablet core, lozenge B with a film coating. The film coating for lozenge B carried a portion of flavoring agents and sweeteners, while the corresponding amount was withdrawn from the lozenge core. Thus the total amount of flavoring agents and sweeteners was the same in both lozenges. The lozenge cores for A and for B had the same composition except for the amounts of flavoring and sweetening agent

The result showed that rating of off-taste and disturbing sensation gave lower score on a five grade scale through the whole test (after 30 seconds, 2 minutes and 5 minutes of testing) for formulation B than for formulation A. The overall liking was higher for formulation B than for formulation A through the whole test. All study persons tested both formulations with at least 15 minutes between the tests. The scale used for off taste and disturbing sensation was a 5-point intensity scale.

## Claims

1. A solid pharmaceutical dosage form for oral administration comprising:
(a) a core comprising at least one active pharmaceutical ingredient (API) selected from the group consisting of anesthetics, antibiotics, anti-inflammatory drugs, anti-migraine drugs, antiseptics, anti-diarrhea drugs, decongestants, erectile dysfunction drugs, mucolytics, muscle relaxants, anti-allergy drugs, stimulants, substances for treatment of oral malodour, antihistamines, expectorants and cough suppressants, wherein the core of the solid dosage form has a weight from 50 mg to 2000 mg, and
(b) a film coating having a weight of from 1% to 15% of the weight of the core, comprising at least one film-forming polymer wherein the at least one film-forming polymer comprises hydroxypropyl methylcellulose, at least one flavoring agent, and at least one sweetener, wherein the film coating is devoid of any API,
wherein the solid pharmaceutical dosage form comprises at least two APIs,
wherein the solid pharmaceutical dosage form optionally comprises one or more inactive component,
wherein said solid pharmaceutical dosage form is selected from the group consisting of a lozenge, a sublingual tablet, a buccal tablet and an orally disintegrating tablet, whereby upon administration the API is capable of being completely dissolved in the oral cavity,
wherein one or more organoleptically disturbing sensations induced by one or more of the APIs and/or of inactive components of the solid pharmaceutical dosage form is/are reduced by constituents of said film coating,
and wherein said dosage form does not contain nicotine.

2. The solid pharmaceutical dosage form according to claim 1, wherein the at least one API is selected from the group consisting of benzocaine, cocaine, dyclonine, lidocaine, amphotericin, chlorotetracycline, domiphen bromide, doxycycline, gramicidin, minocycline, natamycin, neomycin, tetracycline, tyrothricin, acetyl salicylic acid, amlexanox, becaplermin, benzydamine, dexamethasone, flurbiprofen, ibuprofen, paracetamol, triamcinolone, rizatriptan, sumatriptan, zolmitriptan, ambazone, benzoxonium chloride, cetrimonium chloride, cetylpyridinium chloride, chlorhexidine, clotrimazole, hexamidine, hexetidine, hexylresorcinol, metronidazole, miconazol, oxyquinoline, tibezonium iodide, phenylephrine, loperamide, racecadotril, sildenafil, acetylcysteine, ambroxol, bromhexin, carbocysteine, domiodol, eprazinon, etosteine, sobrerol, stepronin, methocarbamol, diphenhydramine, caffeine, zinc salts, meclozine, chlorphenamine maleate, pheniramine maleate, cetirizine, guaifenesin, guaietolin, dropropizine, and dextromethorphan.

3. The solid pharmaceutical dosage form according to claims 1-2, wherein the at least one API is selected from the group consisting of anesthetics, decongestants and cough suppressants.

4. The solid pharmaceutical dosage form according to claim 3, wherein the at least one API is selected from the group consisting of benzocaine, dyclonine, lidocaine, phenylephrine, dropropizine and dextromethorphan.

5. The solid pharmaceutical dosage form according to any preceding claim, wherein the at least one film coating has a thickness from 10 to 500 microns.

6. The solid pharmaceutical dosage form according to any preceding claim, wherein the one or more flavoring agents are selected from the group consisting of natural or synthetic flavouring or aromatizing agents and combinations thereof.

7. The solid pharmaceutical dosage form according to any preceding claim, wherein the one or more sweeteners are selected from the group consisting of synthetic or natural sugars, and combinations thereof.

8. The solid pharmaceutical dosage form according to any preceding claim, wherein it further comprises one or more plasticizers, and/or one or more surfactants.

9. The solid pharmaceutical dosage form according to claim 8, wherein the plasticizers are selected from the group consisting of glycerol, propylene glycol, polyethylene glycol (PEG 200-6000), triacetin, triethyl citrate, diethyl phtalate, dibutyl phtalate, dibutyl sebacete, acetyltriethyl citrate, acethyltributyl citrate, tributyl citrate and oils/glycerides, and the surfactants are selected from the group consisting of Polyoxyethylene (20) sorbitan monolaurate, Polyoxyethylene (20) sorbitan monopalmitate, Polyoxyethylene (20) sorbitan monostearate, Polyoxyethylene (20) sorbitan monooleate, sodium lauryl sulphate (SLS), and poloxamer surfactants.

10. The solid pharmaceutical dosage form according to any preceding claim, wherein the dosage form is a lozenge.

11. The solid pharmaceutical dosage form according to claim 10, wherein it comprises one first API, being benzocaine and one second API, being chlorhexidine dihydrochloride.

12. The solid pharmaceutical dosage form according to claim 11, wherein a unit dose comprises from 1 mg to 3 mg benzocaine and from 2.5 mg to 7.5 mg chlorhexidine dihydrochloride.

## Patentansprüche

1. Feststoffliche pharmazeutische Darreichungsform zur oralen Verabreichung, die Folgendes umfasst:
(a) einen Kern, der mindestens einen pharmazeutischen Wirkbestandteil (active pharmaceutical ingredient, API) umfasst, welcher aus der Gruppe ausgewählt ist, die aus den Anästhetika, den Antibiotika, den entzündungshemmenden Arzneistoffen, den migränebekämpfenden Arzneistoffen, den Antiseptika, den durchfallbekämpfenden Arzneistoffen, den schleimhautabschwellenden Mitteln, den Arzneistoffen gegen Erektionsstörungen, den schleimlösenden Mitteln, den Muskelrelaxantien, den allergiebekämpfenden Mitteln, den Substanzen zur Behandlung von Mundgeruch, den Antihistaminika, den Hustenlöser und den Hustenstillern besteht, wobei der Kern der feststofflichen Darreichungsform ein Gewicht von 50 mg bis 2.000 mg hat, und
(b) einen Überzug, dessen Gewicht 1 % bis 15 % des Gewichts des Kerns ausmacht, wobei er mindestens ein filmbildendes Polymer, wobei das mindestens eine filmbildende Polymer Hydroxypropylmethylcellulose, mindestens einen Geschmacksstoff und mindestens ein Süßungsmittel umfasst, wobei der Überzug frei von jedwedem API ist,
wobei die feststoffliche pharmazeutische Darreichungsform mindestens zwei API umfasst,
wobei die feststoffliche pharmazeutische Darreichungsform möglicherweise eine oder mehrere Komponenten umfasst, die keine Wirkstoffe darstellen,
wobei die feststoffliche pharmazeutische Darreichungsform aus der Gruppe ausgewählt ist, die aus einer Pastille, einer sublingualen Tablette, einer bukkalen Tablette und einer im Mund zerfallenden Tablette besteht, wobei der API dazu befähigt ist, sich nach der Verabreichung vollständig in der Mundhöhle aufzulösen,
wobei eine oder mehrere organoleptisch störende Empfindungen, wie sie von einem oder mehreren der API und/oder der Komponenten, die keine Wirkstoffe darstellen, verursacht werden, durch Bestandteile des Überzugs verringert wird/werden,
und wobei die Darreichungsform keinerlei Nicotin enthält.

2. Feststoffliche pharmazeutische Darreichungsform gemäß Anspruch 1, wobei der mindestens eine API aus der Gruppe ausgewählt ist, welche aus Benzocain, Kokain, Dyclonin, Lidocain, Amphotericin, Chlortetrazyklin, Domiphenbromid, Doxyzyklin, Gramicidin, Minozyklin, Natamycin, Neomycin, Tetrazyklin, Tyrothricin, Acetylsalicylsäure, Amlexanox, Becaplermin, Benzydamin, Dexamethason, Flurbiprofen, Ibuprofen, Paracetamol, Triamcinolon, Rizatriptan, Sumatriptan, Zolmitriptan, Ambazon, Benzoxoniumchlorid, Cetrimoniumchlorid, Cetylpyridiniumchlorid, Chlorhexidin, Clotrimazol, Hexamidin, Hexetidin, Hexylresorcinol, Metronidazol, Miconazol, Oxychinolin, Tibezoniumiodid, Phenylephrin, Loperamid, Racecadotril, Sildenafil, Acetylcystein, Ambroxol, Bromhexin, Carbocystein, Domiodol, Eprazinon, Etostein, Sobrerol, Stepronin, Methocarbamol, Diphenhydramin, Koffein, Zinksalzen, Meclozin, Chlorphenaminmaleat, Pheniraminmaleat, Cetirizin, Guaifenesin, Guaietolin, Dropropizin und Dextromethorphan besteht.

3. Feststoffliche Darreichungsform gemäß den Ansprüchen 1 bis 2, wobei der mindestens eine API aus der Gruppe ausgewählt ist, welche aus den Anästhetika, den schleimhautabschwellenden Mitteln und den Hustenstillern besteht.

4. Feststoffliche pharmazeutische Darreichungsform gemäß Anspruch 3, wobei der mindestens eine API aus der Gruppe ausgewählt ist, welche aus Benzocain, Dyclonin, Lidocain, Phenylephrin, Dropropizin und Dextromethorphan besteht.

5. Feststoffliche pharmazeutische Darreichungsform gemäß einem beliebigen vorhergehenden Anspruch, wobei der mindestens eine Überzug eine Dicke von 10 bis 500 Mikrometern hat.

6. Feststoffliche pharmazeutische Darreichungsform nach einem beliebigen vorhergehenden Anspruch, wobei der oder die mehreren Geschmacksstoffe aus der Gruppe ausgewählt sind, welche aus natürlichen oder synthetischen geschmacksgebenden oder aromatisierenden Mitteln und deren Kombinationen besteht.

7. Feststoffliche pharmazeutische Darreichungsform nach einem beliebigen vorhergehenden Anspruch, wobei das oder die mehreren Süßungsmittel aus der Gruppe ausgewählt sind, welche aus synthetischen oder natürlichen Zuckerarten und deren Kombinationen besteht.

8. Feststoffliche pharmazeutische Darreichungsform gemäß einem beliebigen vorhergehenden Anspruch, wobei sie weiterhin einen oder mehrere Weichmacher und/oder ein oder mehrere Tenside umfasst.

9. Feststoffliche pharmazeutische Darreichungsform gemäß Anspruch 8, wobei die Weichmacher aus der Gruppe ausgewählt sind, welche aus Glycerin, Propylenglykol, Polyethylenglykol (PEG 200-6000), Triacetin, Triethylcitrat, Diethylphthalat, Dibutylphthalat, Dibutylsebacat, Acetyltriethylcitrat, Acetyltributylcitrat, Tributylcitrat und Ölen/Glyceriden besteht, und die Tenside aus der Gruppe ausgewählt sind, welche aus Polyethylenoxid(20)-Sorbitanmonolaurat, Polyethylenoxid(20)-Sorbitanmonopalmitat, Polyethylenoxid(20)-Sorbitanmonostearat, Polyethylenoxid(20)-Sorbitanmonooleat, Natriumlaurylsulfat (SLS) und Poloxamertensiden besteht.

10. Feststoffliche pharmazeutische Darreichungsform gemäß einem beliebigen vorhergehenden Anspruch, wobei es sich bei der Darreichungsform um eine Pastille handelt.

11. Feststoffliche pharmazeutische Darreichungsform gemäß Anspruch 10, wobei sie einen ersten API, bei welchem es sich um Benzocain handelt, und einen zweiten API umfasst, bei welchem es sich um Chlorhexidin-Dihydrochlorid handelt.

12. Feststoffliche pharmazeutische Darreichungsform gemäß Anspruch 11, wobei eine Einheitsdosis 1 mg bis 3 mg Benzocain und 2,5 mg bis 7,5 mg Chlorhexidin-Dihydrochlorid umfasst.

## Revendications

1. Forme de dosage pharmaceutique solide pour une administration orale comprenant :
(a) un noyau comprenant au moins un ingrédient pharmaceutique actif (API) choisi dans le groupe constitué par des anesthésiants, des antibiotiques, des médicaments anti-inflammatoires, des médicaments anti-migraine, des antiseptiques, des médicaments anti-diarrhée, des décongestionnants, des médicaments pour le dysfonctionnement érectile, des mucolytiques, des relaxants musculaires, des médicaments antiallergiques, des stimulants, des substances pour le traitement de mauvaises odeurs orales, des antihistaminiques, des expectorants et des agents de suppression de la toux, le noyau de la forme de dosage solide possédant un poids de 50 mg à 2 000 mg, et
(b) un revêtement en film possédant un poids allant de 1 % à 15 % du poids du noyau, comprenant au moins un polymère filmogène, l'au moins un polymère filmogène comprenant une hydroxypropylméthylcellulose, au moins un agent d'arôme, et au moins un édulcorant, le revêtement en film étant exempt d'un quelconque API,
la forme de dosage pharmaceutique solide comprenant au moins deux API,
la forme de dosage pharmaceutique solide comprenant éventuellement un ou plusieurs composants inactifs, ladite forme de dosage pharmaceutique solide étant choisie dans le groupe constitué par une pastille, un comprimé sublingual, un comprimé buccal et un comprimé se désintégrant par voie orale, moyennant quoi lors de l'administration, l'API est capable d'être complètement dissous dans la cavité orale,
une ou plusieurs sensations perturbantes de manière organoleptique induites par l'un ou plusieurs parmi les API et/ou des composants inactifs de la forme de dosage pharmaceutique solide étant réduite(s) par des constituants dudit revêtement en film,
et ladite forme de dosage ne contenant pas de nicotine.

2. Forme de dosage pharmaceutique solide selon la revendication 1, l'au moins un API étant choisi dans le groupe constitué par benzocaïne, cocaïne, dyclonine, lidocaïne, amphotéricine, chlorotétracycline, le bromure de domiphène, la doxycycline, la gramicidine, la minocycline, la natamycine, la néomycine, la tétracycline, la tyrothricine, l'acide acétylsalicylique, l'amlexanox, la bécaplermine, la benzydamine, la dexaméthasone, le flurbiprofène, l'ibuprofène, le paracétamol, la triamcinolone, le rizatriptan, le sumatriptan, le zolmitriptan, l'ambazone, le chlorure de benzoxonium, le chlorure de cetrimonium, le chlorure de cétylpyridinium, la chlorhexidine, le clotrimazole, l'hexamidine, l'hexétidine, l'hexylrésorcinol, le metronidazole, le miconazol, 1'oxyquinoline, l'iodure de tibézonium, la phényléphrine, le lopéramide, le racécadotril, le sildénafil, l'acétylcystéine, l'ambroxol, la bromhexine, la carbocystéine, le domiodol, l'éprazinone, l'étostéine, le sobrérol, la stepronine, le méthocarbamol, la diphénhydramine, la caféine, des sels de zinc, la meclozine, le maléate de chlorphénamine, le maléate de phéniramine, la cétirizine, la guaïfénésine, la guaïétoline, la dropropizine, et le dextrométhorphane.

3. Forme de dosage pharmaceutique solide selon les revendications 1 et 2, l'au moins un API étant choisi dans le groupe constitué par des anesthésiants, des décongestionnants et des agents de suppression de la toux.

4. Forme de dosage pharmaceutique solide selon la revendication 3, l'au moins un API étant choisi dans le groupe constitué par la benzocaïne, la dyclonine, la lidocaïne, la phényléphrine, la dropopizine et le dextrométhorphane.

5. Forme de dosage pharmaceutique solide selon une quelconque revendication précédente, l'au moins un revêtement en film possédant une épaisseur de 10 à 500 microns.

6. Forme de dosage pharmaceutique solide selon une quelconque revendication précédente, l'agent ou les agents d'arôme étant choisis dans le groupe constitué par des agents d'arôme ou aromatisants naturels ou synthétiques et des combinaisons correspondantes.

7. Forme de dosage pharmaceutique solide selon une quelconque revendication précédente, l'édulcorant ou les édulcorants étant choisi dans le groupe constitué par des sucres synthétiques ou naturels, et des combinaisons correspondantes.

8. Forme de dosage pharmaceutique solide selon une quelconque revendication précédente, qui comprend en outre un ou plusieurs plastifiants et/ou un ou plusieurs tensioactifs.

9. Forme de dosage pharmaceutique solide selon la revendication 8, les plastifiants étant choisis dans le groupe constitué par le glycérol, le propylèneglycol, un polyéthylène glycol (PEG 200 à 6 000), la triacétine, le citrate de triéthyle, le phtalate de diéthyle, le phtalate de dibutyle, le sébaçate de dibutyle, le citrate d'acétyltriéthyle, le citrate d'acétyltributyle, le citrate de tributyle et des huiles/glycérides, et les tensioactifs étant choisis dans le groupe constitué par un monolaurate de polyoxyéthylène (20) sorbitane, un monopalmitate de polyoxyéthylène (20) sorbitane, un monostéarate de polyoxyéthylène (20) sorbitane, un monooléate de polyoxyéthylène (20) sorbitane, le laurylsulfate de sodium (SLS), et des tensioactifs de type poloxamères.

10. Forme de dosage pharmaceutique solide selon une quelconque revendication précédente, la forme de dosage étant une pastille.

11. Forme de dosage pharmaceutique solide selon la revendication 10, qui comprend un premier API, qui est la benzocaïne et un deuxième API, qui est le dichlorhydrate de chlorhexidine.

12. Forme de dosage pharmaceutique solide selon la revendication 11, une dose unitaire comprenant de 1 mg à 3 mg de benzocaïne et de 2,5 mg à 7,5 mg de dichlorhydrate de chlorhexidine.
